# EUROPEAN PATENT SPECIFICATION

(11) **EP 1 215 998 B1**
(45) Date of publication and mention of the grant of the patent: **24.11.2004**
(21) Application number: 00964629.0
(22) Date of filing: 20.09.2000
(51) Int. Cl.: A61B 5/12

(54) **AUDIOMETER FOR SELF-USE BY A PATIENT**
AUDIOMETER ZUR SELBSTANWENDUNG DURCH EINEN PATIENTEN
AUDIOMETRE DESTINE A UNE UTILISATION AUTONOME PAR LE PATIENT

(30) Priority: 20.09.1999 IT RM990193 U
(43) Date of publication of application: 26.06.2002
(73) Proprietor: M.A.Y.K.O. Optical di Bartolomucci Marisa, 80138 Napoli (IT)
(72) Inventor: BARTOLOMUCCI, Gennaro, Corso Umberto I, 381 I-80138 Napoli (IT)
(74) Representative: Leone, Mario
(86) International application number: PCT/IT2000/000371
(87) International publication number: WO 2001/021071

(56) References cited:
- GB-A- 838 563
- GB-A- 2 261 950
- US-A- 3 745 674
- US-A- 3 974 335
- US-A- 4 107 465
- US-A- 5 197 332

## Description

The present invention relates to an audiometer for self-use by a patient. In particular, this invention refers to an audiometer, to perform audiometric tests, comprising an acoustic signal emitting unit and a hearing unit of said signals, apt to cooperate with a user's ear, acoustically connected to said emitting unit.

An audiometric test is generally performed by having a patient hear a sequence of acoustic tones. Each of these acoustic tones has its own frequency inside the audible frequency field (audio band), with such an intensity so as to be perceivable by human ear, for example 30 dB. If the patient does not perceive a certain tone at the above-mentioned intensity, the intensity itself is gradually increased until the patient perceives the subject tone or the intensity reaches a certain higher threshold. This higher threshold is often called pain threshold and is generally fixed at 120 dB. The tone intensities perceived by the patient for each frequency are recorded in a specific card.

The above-mentioned procedure is generally performed for the right ear and the left ear separately.

Audiometers currently utilized to perform audiometric tests are mainly composed by an acoustic signal emitting unit, connected to a hearing unit of these signals, generally consisting in headphones or bone transducer. Typically, the emitting unit is externally protected by a parallelepiped-shaped case.

During the audiometric test, the patient "wears" the hearing unit, whereas the medical or technical personnel drives the acoustic signal emission by interacting with the audiometer emitting unit. In some cases, an acoustically insulated hearing cabin is also provided, wherein the patient takes his/her seat.

The above-described audiometers of the known art have some considerable disadvantages.

First of all, they allow performing an audiometric test in specialized offices only, due to the need of specialized personnel able to interact with the emitting unit.

Furthermore, these audiometers are not easy to handle. In particular, it is required that the room housing the audiometer be equipped with at least a resting plane sufficiently broad for the emitting unit. This may involve a not negligible room engagement and a consequent dedicated arrangement of the room wherein the audiometer is utilized. These considerations are further emphasized when the audiometer provides the installation of a hearing cabin. As a consequence of all this, not all rooms are suitable for lodging a known-art audiometer.

Furthermore, the above-mentioned circumstances, added to the cost of the audiometer itself, may involve an economic burden not bearable by all consulting rooms. For example, generally these costs are not borne by the local national sanitary service surgeries. However, in these surgeries it is anyway necessary to check hearing, especially of elderly patients. In this case, the audiometric test is replaced by a test based on the perception of words pronounced by the medical doctor himself with different intensities. It is clear that this test is highly subjective and not much reliable.

Furthermore, as a consequence of the fact that the audiometric test can be performed almost in specialized offices only, this test is not included among the ones usually performed in periodical medical check-ups. On the other hand, few people choose addressing to a specialized office just for check-up purpose. In fact, in these offices the audiometric test is expensive for the patient, even because this test is typically performed during a deep visit of the hearing apparatus. All this can be to the detriment of an early diagnosis of hearing pathologies.

US 3,745,674 discloses an apparatus for testing a person as to his/her ability to associate audible sounds with visual scenes. This apparatus comprises a screen wherein the scenes are presented, ear phones to deliver the sound and a control board having switch actuators operable by the person to choose the visual scene corresponding to the sound. The apparatus has a support table for the above components and a chair for the user.

US 4, 107,465 discloses an automatic audiometer system providing the emission of a plurality of fixed frequency signals and having related indicating light emitting diodes.

The technical problem the present invention is based upon is to overcome the inconveniences mentioned above by referring to the known art.

This problem is overcome by an audiometer for performing audiometric test comprising:
- an electronic acoustic signal emitting unit, preset for emitting at least a pre-defined sequence of acoustic signals;
- a hearing unit of said signals, apt to cooperate with a user's ear, acoustically connected to said emitting unit; and
- indicating means of acoustic signal to indicate the type of each acoustic signal of said sequence,
characterised in that the audiometer further comprises
- an essentially column-shaped support system, having a base apt to be rested on the floor and a rod connected to said base, said rod carrying, at its top, said emitting unit and indicating means, so as to allow interaction with the user in an upright position.

The present invention provides some important advantages.

First of all, the emitting unit presetting to emit predetermined sequences of acoustic signals, together with the presence of determining means for determining the outcome of an audiometric test, makes the audiometer of the present invention enable a direct and self-use by an individual (the patient) desiring to perform an audiometric test.

Furthermore, the presence of the above-mentioned support system makes the audiometer potentially little bulky and very compact. This makes it usable in any room, and in particular in chemists'shops and in local units of the national sanitary system.

The audiometer of the present invention therefore may allow performing an audiometric test at low cost for the user.

These advantages facilitate and foster a periodic hearing check-up.

Other advantages and features and application modes of the present invention will be evident from the following detailed description of a preferred embodiment thereof, provided by way of example and not for limitative purposes. The figures of the enclosed drawings will be referred to, wherein:
Fig. 1 shows a front perspective view of an embodiment of the audiometer according to the present invention;
Fig. 2 shows a front view of an enlarged detail of the audiometer of Fig. 1;
Fig. 3 shows a front view of the enlarged detail of Fig. 2, wherein some inner components are indicated; and
Fig. 4 shows a schematic representation of the inner electronic components of the audiometer of Fig. 1.

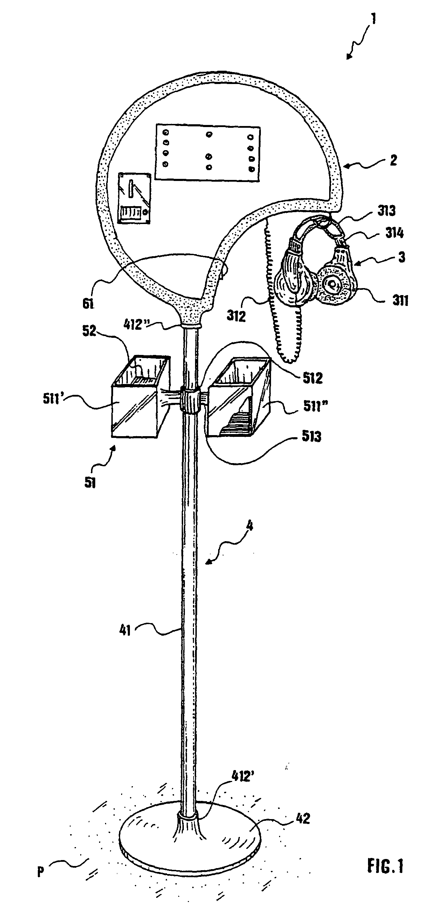

By firstly referring to Fig. 1, an audiometer 1 is preset to perform audiometric tests. To fulfil this function, the audiometer 1 mainly comprises: an emitting unit 2, preset to emit at least a predetermined sequence of acoustic signals; a hearing unit 3 of these acoustic signals, cooperating upon use with a user's ear and acoustically connected to the emitting unit 2; a support system 4 to support the emitting unit 2; and means for determining the outcome of the audiometric test, apt to interact with the user himself.

In the present embodiment, the support system is column-shaped and placed, upon use, on a stamping plane P. In the present context, under "column" the support system 4 having mostly vertical extension is meant.

Furthermore, still in the present embodiment, the above-mentioned determining means comprises indicating means of acoustic signal, to indicate to the user the type of each acoustic signal of said predetermined sequence, and recording cards 52, to record the acoustic signals heard and/or not heard by the user, as it will be illustrated in greater detail hereinafter. Such cards 52 are housed, in the present example, in a card-holder unit 51.

Each of the components introduced above will be now described in greater detail.

The support system 4 comprises a rod 41 and a base 42, secured one to the other by first connecting means of conventional type 412'.

Preferably, the rod 41 and the base 42 will be manufactured with metallic material, to guarantee a better strength of the structure. For example, they can be manufactured with heavy iron and chromium-plated. Furthermore, they can be arranged for the application of advertisement or other type of notice.

In the present embodiment, the base 42 is substantially disc-shaped. Clearly, according to variations of this embodiment it could be differently shaped, for example tripod-like shaped.

First connecting means 412' consists in first threaded joints apt to engage one in the other. These first threaded joins are rigidly connected to a first longitudinal end of the rod 41 and to the base 42, respectively. For example, these threaded joins can be welded on the base 42 and on the rod 41.

A second longitudinal end of the rod 41 is secured, by second connecting means 412", to the emitting unit 2, at a lower end thereof. Also these second connecting means 412" is of conventional type. In particular, these means can be similar to first connecting means 412', and therefore consist of second threaded joints rigidly connected to the second longitudinal end of the rod 41 and to the emitting unit 2, respectively.

As above mentioned, the support system 4 just described confers to the audiometer 1 a mostly vertical extension. This makes the audiometer 1 be little bulky and allows the emitting unit 2 be at the level of the user's trunk.

The arrangement of the set constituted by the emitting unit 2 and by the support system 4 is so that an axis substantially orthogonal to the base 42 passing through the centre thereof, the longitudinal axis of the rod 41 and an axis lying onto a front plane of the emitting unit 2 and passing through the centroid thereof are aligned, for a better balance of the audiometer 1.

Preferably, the rod 41 is about 1.1 m-long, so that the emitting unit 2 is at the level of the trunk of a user in upright position. According to a variant of the present embodiment, the rod 41 may have an adjustable length, so as to adjust to the user's height or to allow even to a sit person to interact with the emitting unit 2.

It is clear that, in other embodiments, alternative configurations of the set constituted by the emitting unit and by the support system, equally well-balanced, may be adopted.

In the present embodiment, the above-mentioned card-holder unit 51 to house cards 52 is secured to the support system 4. This card-holder unit 51 comprises a container formed by two compartments 511' and 511" placed side by side, to contain the cards 52. These compartments 511' and 511 " are substantially parallelepiped-shaped, and preferably made of plastic material. The two-compartment structure guarantees a better stability of the above-mentioned container upon use.

Clearly, in variations of this embodiment, the card-holder unit 51 may have various shapes and comprise any number of compartments. For example, also a compartment housing a pen for filling-in the cards 52 could be provided.

The card-holder unit 51 furthermore comprises securing means for securing the card-holder unit 51 itself to the support system 4, and in particular to the rod 41. In the present embodiment, these securing means comprises a ring 512, which can be inserted onto the rod 41, and a securing screw device (not represented in Figure) of such ring 512. Upon assembling, the ring 512 can be inserted on the rod 41 and made run longitudinally along it until reaching a desired position. Then, the ring 512 can be blocked in this position by means of the above-mentioned securing device.

The cards 52 preferably consist in paper sheets with appropriate pre-printed characters, and are utilized by the user to record the acoustic signals perceived and/or not perceived during the audiometric test. In particular, the cards 52 could be preset to record the perceived acoustic signals only, the not perceived acoustic signals only, or both. According to the present embodiment, they will be preset for separate recordings for the right and the left ear, as it will result more evident from the description of the use thereof in cooperation with the acoustic signal indicating means.

By still referring to Fig. 1, in the present embodiment the hearing unit 3 comprises headphones which can be worn by user. These headphones 3 are preferably provided with two wrapping pavilions 311 and 312, each cooperating, upon wearing the headphones, with a corresponding ear pavilion of the user. To this purpose, each of these two wrapping pavilions 311 and 312 has a half-shell-type shape, so as to fully cover the corresponding ear pavilion and acoustically insulate it from the surrounding environment. These wrapping pavilions 311 and 312 have respective marks (not represented in the Figure) to designate the ear, right or left, whereon the pavilion is to be applied. In particular, the pavilion 311 can cooperate with the left ear, and therefore will be referred to hereinafter as left wrapping pavilion. Similarly, the pavilion 312 will be referred to hereinafter as right wrapping pavilion. The headphones 3 comprise then a strap 313 connecting the two left and right wrapping pavilions 311 and 312. This strap 313 has a substantially semicircular profile, to follow the profile of the user's head. The headphones 3 can also comprise, at the strap 313, means 314 for adjusting the length of the strap 313 itself, to adjust this length to the dimensions of a specific user's head.

Even if the wrapping pavilions 311 and 312 of the headphones 3 provide an acoustic insulation for the ear pavilions, the audiometer according to the present invention will be preferably placed in a separate and little noisy area of the room wherein the audiometer itself is utilized.

It is clear that, according to variants of the present embodiment, the hearing unit may also consist in an earphone or in a loudspeaker incorporated in the emitting unit 2 itself. These variants are consistent with the hitherto described embodiment of the audiometer, even if they could not guarantee an appropriate acoustic insulation of the ear pavilion from the outer environment. The hearing unit 3 may also consist in a single wrapping pavilion of above-described type, which the user puts first near one ear and then near the other one during the audiometric test.

When it is not used, the headphones 3 are housed in a specific seat 31, which receives it so that the headphones themselves are easily removable.

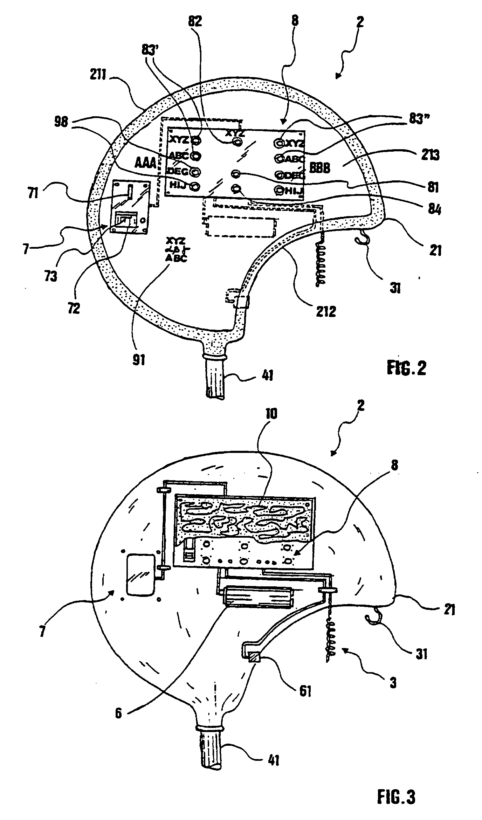

By referring also to Fig. 2, in the present embodiment the seat 31 is obtained by a substantially hook-shaped member. The headphones 3 can therefore be hung up on the hook-shaped member 31 at the strap 313 thereof. This hook-shaped member 31 is secured to the emitting unit 2 according to an arrangement which will be described in greater detail hereinafter.

It is obvious that various alternative shapes for the seat 31 may exist, depending also on the particular manufacturing of the hearing unit 3. For example, the seat 31 could be on the support system 4 instead that on the emitting unit 2, consist in a case instead of a hook, and so on. The headphones 3 could even be simply rested on the upper part of the emitting unit 2.

By still referring to Fig. 2, the emitting unit 2 comprises an outer case 21, containing inside the electronic components of the emitting unit itself.

The outer case 21 is shaped, in the present embodiment, so as to have in front view a substantially half-moon profile. To this purpose, the outer case 21 has a lateral surface with a profile comprising a first substantially convex profile portion 211 and a second substantially concave profile portion 212.

The profile of a front face 213 of the emitting unit 2 therefore recalls the one of a stylized ear. This shape carries out an informative and not only aesthetical function, since it signals to the user the function of the audiometer 1 and draws his/her attention.

The above-mentioned hook-shaped member 31 for the headphones 3 is preferably secured at the second profile portion 212 of the outer case 21. The concavity of this second profile portion 212, in fact, favours the housing of the headphones 3, by protecting it against accidental contacts with outer objects or passing people.

The outer case 21 is advantageously manufactured with a material that allows the adding graphic elements thereon, for example of a serigraphic type. In the present embodiment, these graphic elements comprise writings 91 being the operation instructions of the audiometer.

In the present embodiment, the audiometer 1 also comprises, incorporated in the emitting unit 2, a payment device 7, in particular a common mechanical-type coin box. This box has, at the case 21, a slot 71 to insert coins. These coins will be inserted by a user to start the audiometric test, as it will be better described later. Naturally, the coin box 7 comprises a compartment containing the inserted coins (not represented in the Figures), preferably with a capacity equal or higher than 300 coins.

The coin box 7 may also provide means 72 for returning the inserted coins, which can be automatic or actuatable by a returning key. In particular, the coins possibly returned will come out through an opening 73. This opening 73 could be equipped with a member retaining the returned coins, to avoid that these coins, once come out, fall down onto the stamping plane.

It is clear that, in alternative embodiments, the payment device 7 could even allow inserting banknotes, or provide payment modes alternative to the one here described. Furthermore, this payment device could result absent or disabled whenever payment of charges is not required to perform the audiometric test.

In the present embodiment, the emitting unit 2 is preset to emit a sequence of pure acoustic tones, in particular sinusoidal signals of a predetermined frequency. This sequence provides the generation of four different tones, at the frequencies of 500, 1000, 2000 and 4000 Hz, respectively, which are the most significative ones of the audio band. Each emitted tone has an intensity of 30 dB and lasts 3 seconds. The interval between a tone of the sequence and the following one is 1.

The emitting unit 2 is advantageously preset to send, during performance of an audiometric test, the above-mentioned sequence first to the left ear and then to the right ear. Furthermore, for a greater reliability of the outcome of the audiometric test itself, the emitting unit 2 could repeat several times, for example three times, the above two sequences of four tones.

In the present embodiment, the emitting unit 2 furthermore comprises a set of light indicators, referred to as a whole with the reference numeral 8. Such light indicators 8 are placed at the front face 213 of the outer case 21. These indicators indicate the proceeding status of the audiometric test and the operation condition of the audiometer 1, as it will be illustrated in greater detail later. Nearby each light indicator 8, characters 98 could be provided, for example a serigraphed writing, indicating the role thereof. The status of these light indicators 8, and in particular the on/off condition and/or the colour of the possible irradiated light, is driven by the inner electronics of the emitting unit 2.

In the present embodiment, part of the light indicators 8 represents the above-mentioned indicating means of acoustic signal. In particular, first light indicators of acoustic signal 83' represent first indicating means of acoustic signal, corresponding to acoustic tones sent to the left ear, and second indicators of acoustic signal 83" represent second indicating means of acoustic signal, corresponding to acoustic tones sent to the right ear. These first and second light indicators of acoustic signal 83' and 83" turn on in sequence during performance of an audiometric test to indicate the frequency of the acoustic tone emitted during the turn-on period. Coherently with the four acoustic tones forming the above-described predetermined sequence, four first light indicators of acoustic signal 83' and four second light indicators of acoustic signal 83" are provided, arranged in two parallel rows. Nearby each of these light indicators of acoustic signal 83' and 83" a writing 98 of the above-mentioned type could be present, having the acoustic tone frequency corresponding to that light indicator of acoustic signal.

The light indicators of acoustic signal 83' and 83" cooperate, upon use, with the cards 52 recording the outcome of the audiometric test to determine the outcome of the audiometric test, as it will be illustrated in greater detail later by referring to the operating modes of the audiometer 1.

It is to be noted that, according to a variant of the present embodiment, the indicating means of acoustic signal may also include a device for sending verbal communications to the user through the hearing unit 3. In particular, before emitting a certain acoustic tone, an appropriate communication could be sent to indicate the frequency of such acoustic tone. Therefore, this communication could replace or integrate the turning-on of a certain light indicator of acoustic signal 83' or 83".

In the present embodiment, a light indicator of ongoing test 82 indicates, if turned-on, that an audiometric test is ongoing.

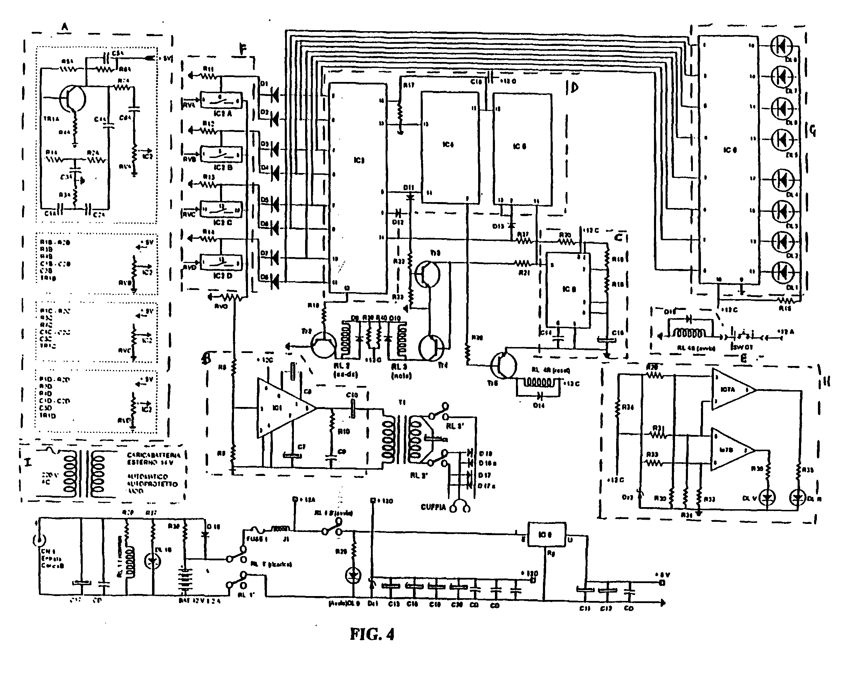

In Fig. 3 the electronic components of the emitting unit 2 contained in the case 21 is schematically illustrated. These components are designed to perform the here-described functions of the audiometer 1. In particular, they allow the emission of acoustic signals, the control of the status of the indicating means of acoustic signal and of the possible other light indicators and the control of the several units forming the audiometer 1 and the interaction thereof. A specific possible manufacturing of these electronic components will be later described in greater detail, by referring to Fig. 4.

By referring to the above-mentioned Fig. 3, in general terms the subject electronic components comprise a system for emitting acoustic signals, designated with 10 as a whole. The system 10 is connected to the hearing unit 3, whereto the acoustic signals are transmitted. The system 10 is also connected to the coin box 7.

The emitting unit 2 furthermore comprises, still within the case 21, a self-power supply unit 6, in particular a rechargeable electric accumulator. Preferably, this accumulator 6 is of a 12V and 1.2A type. Still according to a preferred embodiment, the electric accumulator allows performing about 250 audiometric tests, starting from full charge condition. This accumulator 6 can be connected to an outer recharging unit (not represented in the figures) by a connector 61, placed at the outer case 21 of the emitting unit 2. This recharging unit will typically consist in a common battery-charger well known to the person skilled in the art. Preferably, this battery-charger will be 220/14V and 0.3A type and a thermally protected type. Furthermore, preferably the full recharging operation will take no more than 15 hours, for example 12 hours.

The presence of the above-described self-power supply unit 6 provides a further advantage of the audiometer according to the present invention compared to the known-art audiometers. In fact, these last audiometers are generally powered directly by the external supply mains. On the contrary, the above-described audiometer, upon use, does not require the presence of an external power supply source. Consequently, upon use, it will not have to be necessary placed nearby a power source, in particular nearby an electric outlet. This broadens the variety of possible rooms and locations for the audiometer itself.

Then, the fact that the self-power supply unit is incorporated in the emitting unit allows a compact and little bulky manufacturing.

In the present embodiment, the above-mentioned light indicators 8 also include a level light indicator, designated with 84 in Fig. 2, to indicate the charge level of the accumulator 6. Such level light indicator 84 could be possibly powered by a battery. The level light indicator 84 can have three colours, for example: green, to indicate that the accumulator 6 has a high charge level, and therefore allows performing a quite high number of audiometric tests; orange, to indicate that the accumulator 6 is almost exhausted, and therefore allows performing a quite low number of audiometric tests; and red, to indicate that the accumulator 6 is exhausted, and therefore does not allow performing any audiometric test. The inner electronics of the emitting unit 2 controls the status of the accumulator 6, and consequently drives the colour of the light emitted by this level light indicator 84.

In the present embodiment, the light indicators 8 further include a recharge light indicator, designated with 81 in Fig. 2, to indicate that the accumulator 6 is being recharged.

For sake of completeness, Fig. 4 shows a schematic representation of an embodiment example of the inner electronics of the emitting unit 2. A list of the components represented in this last Figure, together with preferred values of the corresponding parameters, is reported in the following table.

In particular, Fig. 4 shows an electric diagram, constituted by the following stages:
a) 4 sinusoidal oscillators of adjustable amplitude;
b) 1 low-power amplifier with fixed gain and adjustable output amplitude;
c) 1 square-wave oscillator stage;
d) 3 divider stages;
e) 1 bistable relais on/off stage;
f) 4 electronic switches;
g) 1 driver;
h) circuit to control battery status; and
i) battery charger;

| | | | |
|---|---|---|---|
| R1A-R2A | 33KΩ 1/8W 1% | C1D-C2D | 1nF 63V pol. 5% |
| RIB-R2B | 33KΩ 1/8W 1% | C3A | 47nF 63V pol. 5% |
| R1C-R2C | 33KΩ 1/8W 1% | C3B | 22nF 63V pol. 5% |
| RID-R2D | 33KΩ 1/8W 1% | C3C | 10nF 63V pol. 5% |
| R3A | 7,5KΩ 1/8W 1% | C3D | 4,7nF 63V pol. 5% |
| R3B | 9KΩ 1/8W 1% | C4A-B-C-D | 220nF 63V pol. 5% |
| R3C | 12KΩ 1/8W 1% | C5A-B-C-D | 470pF 50V disc |
| R3D | 15KΩ 1/8W 1% | C6A-B-C-D | 220nF 63V pol. 10% |
| R4A | 130KΩ 1/8W 1% | C7 | 4,7 µF 25V elect. |
| R4B | 120KΩ 1/8W 1% | C8(eliminated) | 1 µF 25V elect. |
| R4C | 100KΩ 1/8W 1% | C9-14-16 | 100 nF 63V pol.10% |
| R4D | 56KΩ 1/8W 1% | C10 | 220 µF 25V elect. |
| R5A-B-C-D | 1MΩ 1/8W 1% | C11-12-13-CX | 100 µF 25V elect. |
| R6A-B-C-D | 4,7KΩ 1/8W 1% | C17-18-19-20 | 100 µF 25V elect. |
| R7A-B-C-D | 10KΩ 1/8W 1% | C15 | 10 µF 25V elect. |
| R8 | 10KΩ 1/8W 1% | CDx4 | 100 nF 50V pellet |
| R9 | 4,7KΩ 1/8W 1% | TR1A-B-C-D | BC549B |
| R10 | 10Ω 1/8W 1% | TR2-3-4-5 | BC549B |
| R11-12-13-14 | 68KΩ 1/8W 1% | | |
| R15 | 1KΩ 1/8W 1% | IC 1 | LM386N |
| R16 | 1KΩ 1/8W 1% | IC 2 | CD4066 |
| R17 | 68KΩ 1/8W 1% | IC 3-4-5 | CD4017 |
| R18 | 172KΩ 1/8W 1% | IC 6 | UNL2804A |
| R19 | 270KΩ 1/8W 1% | IC 8 | NE555N |
| R20 | 4,7KΩ 1/8W 1% | IC 7 | LM358N |
| R21 | 68KΩ 1/8W 1% | IC 9 | 78L05 |
| R22 | 4,7KΩ 1/8W 1% | | |
| R23 | 33KΩ 1/8W 1% | D1./.D17, D17a-b | 1N4148 |
| R24 | 680KΩ 1/8W 1% | D18 | 1N4007 |
| R25 | 1KΩ 1/8W 1% | DZ 1 | 13V 1W |
| R26 | 1KΩ 1/8W 1% | DZ 2 | 6,2V 1W |
| R27 | 68KΩ 1/8W 1% | RL 1 | Pelais 12V 1A 2sc. |
| R28 | 82Ω 0.5W 5% | RL 2 | Reed Relais 6V 1 com. |
| R29 | 10KΩ 1/8W 1% | RL 3 | Reed Relais 6V 1 com. |
| R30 | 10KΩ 1/8W 1% | RL 4 S-R | Relais 12V 1A 2s bist. |
| R31 | 22KΩ 1/8W 1% | | |
| R32 | 10KΩ 1/8W 1% | DL 1./.4 | LED 8mm orange |
| R33 | 10,3KΩ 1/8W 1% | DL 5./.8 | LED 8mm yellow |
| R34 | 10KΩ 1/8W 1% | DL V-R | LED 5mm green-red |
| R35 | 1KΩ 1/8W 1% | DL 9 | LED 8mm green |
| R36 | 1KΩ 1/8W 1% | DL 10 | LED 5mm green |
| R37 | 1KΩ 1/8W 1% | | |
| R38 | 12KΩ 0,5W 5% | F1 | 315 mA T Fuse |
| R39-40 | 82Ω 0,5W 5% | J1 | Induct. 60 µH |
| RVA-B-C-D | Trimmer 22K | T1 | Transf. Imp. 1:1 |
| RV G | Trimmer 10K | AL | Automatic battery recharger Italtras mod.PK12V 05 |
| C1A-C2A | 10nF 63V pol. 5% | | |
| C1B-C2B | 4,7nF 63V pol. 5% | HEADPHONES | 2 x 30 Ω (Philips SBC HP100) |
| C1C-C2C | 2,2nF 63V pol. 5% | Batt. Rec. Pb | 12 V 1,2 A |

The operation of the stages a) - i) is briefly illustrated herebelow.

As far as stage a) is concerned, the four oscillators TR1A-TR1D generate a pure sinusoidal wave with frequency depending upon the value of the components designated with R1 A/D, R2 A/D, C3 A/D, C1 A/D, C2 a/d, R3 A/D here calculated so as to generate four frequencies of: 500 Hz - 1000 Hz - 2000 Hz - 4000 Hz. The output amplitude, adjustable by RvAöD, is adjusted so that, once a perfectly symmetric sinusoid is generated, a signal is provided apt to reproduce a 30dB sound intensity in the headphones, for a predetermined adjustment of the amplifier stage (RVG).

As far as stage b) (amplifier stage) is concerned, constituted by IC1 (LM386), the gain thereof is kept relatively low to obtain low distortion. The output thereof is connected to the primary of an audio coupling transformer with ratio 1:1, so as to fully insulate the acoustic transducer of the electronic circuits, to comply with safety regulations. The switch performed by the contacts of RL2 connected to the secondary of this transformer commute the generated acoustic tones, alternatively to the right and left pavilion. RL3 interrupts the headphones connection for the whole time exceeding 3 seconds of note hearing; this allows obtaining a perfect silence in headphones during tone change.

As far as the stage c) (oscillator stage) is concerned, constituted by IC8 (NE555), it is designed to generate a square wave with asymmetric ratio (high status H=3" ; low status L=1,2").

As far as the subsequent stages d) to f) are concerned, they will be now examined all together. In standby condition, the circuit is wholly not powered (almost null power absorption). Only upon inserting a new coin, the coin-box switch powers temporarily (for 300 ms) the "set" coil of RL4 which is a set/reset-type bistable relais. This causes RL4S' closing and the whole circuit powering (LD9 turned-on).

Once the circuit is powered, the integrated circuits IC 3-4-5 are immediately reset and enabled for operating.

The four sinusoidal oscillators start working, but all the electronic switches are still open. The pin 12 of IC3 is in an H status, therefore RL2 is powered.

The integrated circuit IC8A-B controls the charging status of the accumulator (if voltage on pin 5 is greater than voltage on pin 6 the led is green; if voltage on pin 2 is lower than voltage on pin 3 the led becomes red).

The integrated circuit IC7 generates a square wave of 3 H and 1.2 L (the first positive pulse lasts about twice). In this step only IC5 can count: in fact the pin 3 of IC3 after the reset is in an H status and therefore by means of Tr3 stops pulses sent on Tr4 base, causing the cut-off thereof. Also the pin 7 of IC5, being still in a L status, stops pulses sent on pin 14 of IC3.

As soon as the positive edge of the third pulse of IC7 starts, the pin 5 of IC5 reaches a H status and therefore it will reach the pin 14 of IC3 too, which could then count. In this step, therefore, the following takes place contemporarily:
- the pin 2 of IC3 reaches a H status and therefore the 500-Hz tone generated by Tr1A could reach IC1 to be amplified;
- the pin 3 of IC3 by reaching a L status will allow activating IC3 for the whole duration of the positive pulse generated by IC7;
- the pulses generated by IC7 make the IC3 count (pin 2-4-7-10) advance in sequence; and
- the fifth pulse brings to an L status the pin 12 of IC3 which, by deactivating RL2, will commute the audio output on the right headphone earphone. The same four tones will repeat then again (pin 5-6-9-11 of IC3 to H).

Actually, the outputs of IC3 switch only during clock rising edges (pin 14) and therefore the variation thereof takes place only after a whole clock cycle (time of halfwave at H status + time of halfwave at L status = 4.2 seconds), which would cause silence absence between one tone and the other; to avoid this, RL3 connects the earphones only during the positive halfwave generated by IC7 and therefore for only 3 seconds by inserting a 1.2 silence between two tones.

By passing from left to right, since the output related to the counting of the fifth pulse (pin 1 of IC3) has been skipped, the silence increases by 5 and the pin 1 itself stops the tone emission by activating Tr3. All the outputs of IC3 driving the electronic switches also determine, by means of IC6, the turning-on of the corresponding signalling leds (DL1-DL8).

IC3 repeats the whole counting cycle (from out 0 to out 9) for three times. As soon as the fourth cycle starts, the pin 3 reaches 1, therefore the pin 7 of IC4 by reaching a H status will activate the "reset" coil of RL4 which will cause the opening of the RL4S' contact and the turning-off of the whole cycle (end of test).

As far as stage h) is concerned, the integrated circuit IC8AB signals the status of the inner accumulators. If battery tension is lower than 9 Volt, the red led DLR turns on. Under efficiency conditions the green led DLV is turned on.

The inner accumulator is being recharged, by the relative recharger, in about 10 hours; during this period, once the mains cable is inserted, the remaining circuit is wholly insulated from any inner or outer voltage for the RL1 switching. Only the battery charger remains operating.

As far as stage i) is concerned at last, the battery charger is a typical electronic circuit apt to recharge the above-described Pb accumulator, housed in a specific container making a separate unit which can be connected to the audiometer through the connector 61 and to the 220V mains through usual AC cable.

From what has been so far described, it will be appreciated that the audiometer according to the present invention is likely to be manufactured in modules and therefore it can be easily assembled/disassembled. This constitutes a further advantage of the present invention, also because this module-manufacturing makes transport easier.

In particular, based on the present embodiment, it will be sufficient performing the following procedures to mount the audiometer 1:
- to connect the first end of the rod 41 to the base 42 by the first connecting means 412';
- to secure the card-holder unit 51 to the rod 41; and
- to connect the second end of the rod 41 to the emitting unit 2 by the second connecting means 412".

During the first installation of the audiometer 1, a charging cycle of the electric accumulator could be necessary. Once these procedures are finished, the audiometer 1 will be ready to start.

It will be appreciated that the above-mentioned procedures are extremely easy and can be performed by non-technical personnel. This enables the installation and the utilization of the audiometer according to the present invention in non-specialized centres, and in particular in chemists'shops, boarding-houses and in suburban surgeries of national sanitary system.

Based on the hereto provided description, it will be also appreciated that the audiometer according to the present invention is potentially little bulky. Preferred sizes of the audiometer are 1.61 m in height, 0.50 m in width and 0.16 m in depth. Furthermore, if the coin-containing compartment is empty, it will preferably weigh no more than 12 kg.

The operating modes of the audiometer 1 will be now illustrated in greater detail.

The audiometer 1 usually is in a stand-by condition, usually called "standby" condition, during which it is ready to perform the audiometric test. The audiometer 1 may pass from the standby condition to the operating condition of performing an audiometric test as a consequence of an appropriate stimulating action by a user. In the present embodiment, this stimulating action consists in inserting the requested coins in the slot 71 of the coin box 7.

Once the above-mentioned stimulation is received, the coin box 7 checks whether type and number of the inserted coins are those required to perform the audiometric test. If this check is positive, the emitting unit starts the audiometric test. If this check is negative, the coin box 7 retains the inserted coins, which will be returned by the returning means 72. This return could take place automatically or by actuating an appropriate button, as already illustrated.

According to a variant of the present embodiment, if payment of charge were not necessary to perform the audiometric test, the above stimulating action could be carried out by a control device, a button for example. This could take place, for example, if the audiometer according to the present invention is utilized in a surgery.

Naturally, the audiometric test could be actually performed provided that the charge level of the accumulator 6 is enough to perform the test itself. This charge level is signalled to the user by the level light indicator 84. The audiometer will return the coins possibly inserted by mistake by the user in the coin box 7 when the accumulator 6 is exhausted (red colour light emitted by the level light indicator 84).

At this point, the user takes the headphones 3 from their seat 31 and wears them, so that the two left and right wrapping pavilions 311 and 312 well cover the corresponding ear pavilions, so as to acoustically insulate them.

The time interval elapsing from the coin acceptation and the audiometric test starting, in the present embodiment, is predetermined and equal to 10. In a variant of this embodiment, such time interval could be established by the user, for example by pushing a starting-test button. According to another variant of execution, a sensor could be provided, incorporated in the seat 31 of the headphones 3, to signal to the emitting unit 2 the taking of the headphones 3 themselves.

The test starts with the emission by the emitting unit 2, a first sequence of four acoustic tones of the already described type. This first sequence is transmitted to the left wrapping pavilion 311 of the headphones 3. The emitting of a certain tone transmitted to the left ear is signalled to the user by the turning-on of a respective light indicator of the first light indicators of acoustic signal 83' on the outer case 21.

Once a certain time interval has elapsed since the emission of this first sequence for example 4 seconds, the emitting unit 2 emits a second sequence of acoustic tones, identical to the previous one, which is transmitted to the right wrapping pavilion 312 of the headphones 3. Similarly to the previous case, the emission of a tone at a certain frequency is signalled to the user by the turning-on of the respective light indicator of the second light indicators of acoustic signal 83".

At the same time, with the above-mentioned emission of acoustic tones, the user will record on the card 52 the perception or not perception of a certain tone.

Therefore, the cooperation of the light indicators of acoustic signal 83', 83" with the recording cards 52 allows determining the outcome of the audiometric test.

Once the emission of acoustic signals is finished, the audiometer returns to the standby condition and the user puts the headphones 3 back into the seat 31.

It will be appreciated that the present invention provides the advantage of allowing an easy and immediate analysis of the audio band in the auditive sensibility. It will be also appreciated that the operation of the audiometer according to the present invention is very easy.

In the present embodiment, an audiometric test not deep and for explorative or check-up purposes only is provided. This type of audiometric test is coherent with using the audiometer of the present invention in chemists' shops, and with handling the test by the patient himself. Should this test point out that some frequencies are not perceived, the user could choose to undergo a deeper examination, in a specialized centre.

It is clear that, in variants of this embodiment, the number, intensity and nature of the acoustic signals and of the frequencies emitted by the emitting unit could be different with respect to what has been hereto described. In particular, the emitting unit could be preset to perform a deeper audiometric test, which better identifies type and level of the possible hearing deficiency. Such a deeper analysis is clearly more effective and pertinent if the audiometer according to the present invention is utilized in a surgery or in another organization of the sanitary system.

Furthermore, the audiometer according to the present invention could provide performing two or more different audiometric tests, for example an explorative one only and another one deeper, by user's choice.

It is to be observed that the number, the arrangement and the role of the light indicators 8 could vary according to the requirements of specific embodiments of the audiometer according to the present invention. In particular, additional light indicators could be provided to indicate possible damage or operating anomalies of the audiometer, or the need to empty the coin-containing compartment.

According to a further variant of the present embodiment, the card-holder unit could be incorporated inside the emitting unit and not accessible by user. According to this variant, the emitting unit will comprise also a printing unit, to print on specific cards the outcome of the audiometric test. In this case, the determining means could comprise also a button, through which the user interacts with the emitting unit to indicate the perceived and/or not perceived acoustic signals.

The present invention has been so far described according to an embodiment thereof. It is to be meant that this preferred embodiment can be subject to variants belonging to the same inventive core, all of them within the protection scope of the herebelow reported claims.

## Claims

1. An audiometer (1) for performing audiometric tests comprising:
- an electronic acoustic signal emitting unit (2), preset for emitting at least a pre-defined sequence of acoustic signals;
- a hearing unit (3) of said signals, adapted to cooperate with a user's ear, acoustically connected to said emitting unit; and
- determing means (52, 83', 83") for determining the outcome of audiometric test, adapted to interact with the user himself, comprising indicating means of acoustic signal (83', 83") to indicate the type of each acoustic signal of said sequence,
**characterised in that** the audiometer further comprises
- an essentially column-shaped support system (4), having a base (42) adapted to be rested on the floor (P) and a rod (41) connected to said base, said rod carrying, at its top, said emitting unit and indicating means, so as to allow interaction with the user in an upright position.

2. The audiometer (1) according to claim 1, wherein said indicating means of acoustic signal comprises light indicators of acoustic signal (83', 83").

3. The audiometer (1) according to claim 1 or 2, wherein said indicating means of acoustic signal comprises first (83') and second (83") indicating means, to indicate the type of each acoustic signal sent to left ear and to right ear, respectively.

4. The audiometer (1) according to any of the preceding claims, comprising cards (52) for recording the outcome of the audiometric test.

5. The audiometer (1) according to any of the preceding claims, comprising a card-holder unit (51) to house cards (52) for recording the outcome of the audiometric test.

6. The audiometer (1) according to claim 5, wherein said card-holder unit (51) has means for securing to said support system (4).

7. The audiometer (1) according to claim 5 or 6, wherein said card-holder unit (51) comprises a container having at least two compartments (511', 511") to contain said cards (52).

8. The audiometer (1) according to any of the preceding claims, comprising a self-power unit (6) of said emitting unit (2).

9. The audiometer (1) according to claim 8, wherein said self-power unit (6) is housed into said emitting unit (2).

10. The audiometer (1) according to claim 8 or 9, wherein said self-power unit comprises a rechargeable electric accumulator (6).

11. The audiometer (1) according to claim 10, comprising:
- a level light indicator (84), to indicate the charge level of said accumulator (6); and
- a recharge light indicator (81), to indicate that said accumulator (6) is being recharged.

12. The audiometer (1) according to any of the preceding claims, wherein said at least pre-defined sequence comprises pure acoustic tones.

13. The audiometer (1) according to any of the preceding claims, wherein said emitting unit (2) comprises an outer case (21), having a front profile essentially half-moon shaped, said profile comprising a first substantially convex profile portion (211) and a second substantially concave profile portion (212).

14. The audiometer (1) according to any of the preceding claims, comprising a substantially hook-shaped member (31), being a seat for said hearing unit (3).

15. The audiometer (1) according to claims 13 and 14, **characterized in that** said hook (31) is housed at said second profile portion (212) of said case (21).

16. The audiometer (1) according to any of the preceding claims, comprising a payment device (7).

## Patentansprüche

1. Audiometer (1) zum Durchführen von audiometrischen Prüfungen, mit:
- einer elektronischen Emittiereinheit (2) für akustische Signale, die so voreingestellt ist, dass sie wenigstens eine vorbestimmte Sequenz von akustischen Signalen emittiert;
- einer Höreinheit (3) für die Signale, die dafür ausgebildet ist, mit einem Ohr des Benutzers zusammenzuwirken, und mit der Emittiereinheit akustisch verbunden ist; und
- einer Bestimmungseinrichtung (52, 83', 83") zum Bestimmen des Ergebnisses der audiometrischen Prüfung, die dafür ausgebildet ist, mit dem Benutzer selbst in Dialog zu treten, beinhaltend
- eine Anzeigeeinrichtung für akustische Signale (83', 83") zum Anzeigen des Typs jedes akustischen Signals der Sequenz,
**dadurch gekennzeichnet, dass** das Audiometer weiter aufweist
- ein im wesentlichen säulenförmiges Tragsystem (4), das einen Fuß (42) hat, der dafür ausgebildet ist, auf dem Boden (P) zu ruhen, und eine Stange (41), die mit dem Fuß verbunden ist, wobei die Stange an ihrem oberen Ende die Emittiereinheit und die Anzeigeeinrichtung trägt, um so den Dialog mit dem Benutzer in einer aufrechten Position zu erlauben.

2. Audiometer (1) nach Anspruch 1, wobei die Anzeigeeinrichtung für akustische Signale eine Lichtanzeigevorrichtung für akustische Signale (83', 83") aufweist.

3. Audiometer (1) nach Anspruch 1 oder 2, wobei die Anzeigeeinrichtung für akustische Signale eine erste (83') und eine zweite (83") Anzeigeeinrichtung aufweist, um den Typ jedes akustischen Signals anzuzeigen, das zu dem linken Ohr bzw. zu dem rechten Ohr gesendet wird.

4. Audiometer (1) nach einem der vorhergehenden Ansprüche, mit Karten (52) zum Aufzeichnen des Ergebnisses der audiometrischen Prüfung.

5. Audiometer (1) nach einem der vorhergehenden Ansprüche, mit einer Kartenhaltereinheit (51) zum Aufnehmen von Karten (52) zum Aufzeichnen des Ergebnisses der audiometrischen Prüfung.

6. Audiometer (1) nach Anspruch 5, wobei die Kartenhaltereinheit (51) eine Einrichtung zum Befestigen an dem Tragsystem (4) hat.

7. Audiometer (1) nach Anspruch 5 oder 6, wobei die Kartenhaltereinheit (51) einen Behälter aufweist, der wenigstens zwei Fächer (511', 511") zum Aufnehmen der Karten (52) hat.

8. Audiometer (1) nach einem der vorhergehenden Ansprüche, mit einer Eigenstromversorgungseinheit (6) für die Emittiereinheit (2).

9. Audiometer (1) nach Anspruch 8, wobei die Eigenstromversorgungseinheit (6) in der Emittiereinheit (2) untergebracht ist.

10. Audiometer (1) nach Anspruch 8 oder 9, wobei die Eigenstromversorgungseinheit einen wiederaufladbaren elektrischen Akkumulator (6) aufweist.

11. Audiometer (1) nach Anspruch 10, mit:
- einer Ladezustandslichtanzeigevorrichtung (84) zum Anzeigen des Ladezustands des Akkumulators (6); und
- einer Wiederaufladungslichtanzeigevorrichtung (81) zum Anzeigen, dass der Akkumulator (6) wieder aufgeladen wird.

12. Audiometer (1) nach einem der vorhergehenden Ansprüche, wobei die wenigstens eine vorbestimmte Sequenz reine akustische Töne umfasst.

13. Audiometer (1) nach einem der vorhergehenden Ansprüche, wobei die Emittiereinheit (2) ein äußeres Gehäuse (21) aufweist, das ein Frontprofil hat, welches im Wesentlichen halbmondförmig ist, wobei das Profil einen ersten, im Wesentlichen konvexen Profilteil (211) und einen zweiten, im Wesentlichen konkaven Profilteil (212) aufweist.

14. Audiometer (1) nach einem der vorhergehenden Ansprüche, mit einem im Wesentlichen hakenförmigen Teil (31), das einen Sitz für die Höreinheit (3) bildet.

15. Audiometer (1) nach den Ansprüchen 13 und 14, **dadurch gekennzeichnet, dass** der Haken (31) in dem zweiten Profilteil (212) des Gehäuses (21) untergebracht ist.

16. Audiometer (1) nach einem der vorhergehenden Ansprüche, mit einer Zahlvorrichtung (7).

## Revendications

1. Audiomètre (1) pour réaliser des tests audiométriques comportant :
- une unité d'émission de signaux acoustiques électroniques (2), préréglée pour émettre au moins une séquence prédéfinie de signaux acoustiques,
- une unité d'audition (3) desdits signaux, adaptée pour coopérer avec l'oreille d'un utilisateur, acoustiquement reliée à ladite unité d'émission, et
- des moyens de détermination (52, 83', 83") pour déterminer le résultat d'un test audiométrique, adaptés pour interagir avec l'utilisateur lui-même, comportant des moyens d'indication de signal acoustique (83', 83") pour indiquer le type de chaque signal acoustique de ladite séquence,
**caractérisé en ce que** l'audiomètre comporte en outre :
- un système de support essentiellement en forme de colonne (4), ayant une base (42) adaptée pour être fixée sur le sol (P) et une tige (41) reliée à ladite base, ladite tige portant, à son bout, ladite unité d'émission et lesdits moyens d'indication, de manière à permettre l'interaction avec l'utilisateur dans une position debout.

2. Audiomètre (1) selon la revendication 1, dans lequel lesdits moyens d'indication de signal acoustique comportent des indicateurs lumineux de signal acoustique (83', 83").

3. Audiomètre (1) selon la revendication 1 ou 2, dans lequel lesdits moyens d'indication de signal acoustique comportent des premier (83') et second (83") moyens d'indication, pour indiquer le type de chaque signal acoustique envoyé à l'oreille gauche et à l'oreille droite, respectivement.

4. Audiomètre (1) selon l'une quelconque des revendications précédentes, comportant des cartes (52) pour enregistrer le résultat du test audiométrique.

5. Audiomètre (1) selon l'une quelconque des revendications précédentes, comporte une unité porte-cartes (51) pour y insérer des cartes (52) destinées à enregistrer le résultat du test audiométrique.

6. Audiomètre (1) selon la revendication 5, dans lequel ladite unité porte-cartes (51) a des moyens pour être fixée sur ledit système de support (4).

7. Audiomètre (1) selon la revendication 5 ou 6, dans lequel ladite unité porte-cartes (51) comporte un conteneur ayant au moins deux compartiments (511', 511") pour contenir lesdites cartes (52).

8. Audiomètre (1) selon l'une quelconque des revendications précédentes, comportant une unité à alimentation propre (6) de ladite unité d'émission (2).

9. Audiomètre (1) selon la revendication 8, dans lequel ladite unité à alimentation propre (6) est insérée dans ladite unité d'émission (2).

10. Audiomètre (1) selon la revendication 8 ou 9, dans lequel ladite unité à alimentation propre comporte un accumulateur électrique rechargeable (6).

11. Audiomètre (1) selon la revendication 10, comportant :
- un indicateur lumineux de niveau (84), pour indiquer le niveau de charge dudit accumulateur (6), et
- un indicateur lumineux de recharge (81), pour indiquer que ledit accumulateur (6) est en cours de recharge.

12. Audiomètre (1) selon l'une quelconque des revendications précédentes, dans lequel ladite au moins une séquence prédéfinie comporte des tonalités acoustiques pures.

13. Audiomètre (1) selon l'une quelconque des revendications précédentes, dans lequel ladite unité d'émission (2) comporte un boîtier extérieur (21), ayant un profil avant ayant sensiblement une forme de demi-lune, ledit profil comportant une première partie de profil sensiblement convexe (211) et une seconde partie de profil sensiblement concave (212).

14. Audiomètre (1) selon l'une quelconque des revendications précédentes, comportant un élément ayant sensiblement une forme de crochet (31), étant un support pour ladite unité d'audition (3).

15. Audiomètre (1) selon la revendication 13 et 14, **caractérisé en ce que** ledit crochet (31) est placé au niveau de ladite seconde partie de profil (212) dudit boîtier (21).

16. Audiomètre (1) selon l'une quelconque des revendications précédentes, comportant un dispositif de paiement (7).
